# EUROPEAN PATENT SPECIFICATION

(11) **EP 4 349 817 B1**
(45) Date of publication and mention of the grant of the patent: **23.04.2025**
(21) Application number: 23158350.1
(22) Date of filing: 23.02.2023
(51) Int. Cl.: C07D 251/54, C07D 251/60

(54) **MELAMINE PROCESS WITH A TWO-STAGE PURIFICATION OF MELAMINE OFFGAS**
MELAMIN-VERFAHREN MIT ZWEISTUFIGER AUFREINIGUNG VON MELAMIN-ABGAS
PROCÉDÉ DE PRODUCTION DE MÉLAMINE AVEC PURIFICATION EN DEUX ÉTAPES DE GAZ DE DÉGAGEMENT DE MÉLAMINE

(30) Priority: 07.10.2022 EP 22200402
(43) Date of publication of application: 10.04.2024
(73) Proprietor: CASALE SA, 6900 Lugano (CH)
(72) Inventor: GAMBA, Simone, 24040 Pagazzano (BG) (IT)
(74) Representative: M. Zardi & Co S.A.

(56) References cited:
- WO-A1-2024/083571
- US-A1- 2003 028 020
- US-B2- 7 311 759
- DUNET GUILLAUME: "Written opinion of WO2024083571", 8 August 2024 (2024-08-08), pages 1 - 6, XP093198099, Retrieved from the Internet <URL:https://register.epo.org/application?number=EP23158350&lng=en&tab=doclist>
- GAMBA SIMONE: "CASALE LEM TM IMPROVED PROCESS: KNOW-HOW AND TECHNOLOGY, THE BEST MIX TO MAXIMIZE ENVIRONMENTAL SUSTAINABILITY", NITROGEN + SYNGAS 2022 INTERNATIONAL CONFERENCE, 29 March 2022 (2022-03-29), pages 1 - 30, XP093198361
- DRENNAN SCOTT ET AL: "Urea Deposits: Risk Assesment or Direct prediction?", 29 March 2022 (2022-03-29), pages 1 - 8, XP093198013, Retrieved from the Internet <URL:https://convergecfd.com/blog/urea-deposits-risk-assessment-or-direct-prediction>

## Description

### Field of application

The invention is in the field of industrial production of melamine. The invention particularly pertains to a high-pressure melamine synthesis process including a two-stage washing of the melamine offgas with urea melt.

### Prior art

Melamine is produced at an industrial scale starting from urea following either a non-catalytic high-pressure (HP) process or a low-pressure (LP) catalytic process. The non-catalytic high-pressure process is considered the most advantageous and is becoming predominant.

In the high-pressure process, a urea melt is reacted at pressure which is generally above 70 bar, typically 75 to 200 bar. The temperature of reaction is typically around 375 °C.

The melamine synthesis section may include two reactors arranged in series wherein the first reactor produces a raw melamine melt and the second reactor removes carbon dioxide from the raw melamine melt using a stripping agent such as ammonia. Said second reactor may be termed post-reactor or stripping reactor. The first reactor and the second reactor may be separate vessels or combined in a single apparatus.

The melamine-containing product stream is sent to further treatments, typically in a plant section operating at a lower pressure than the pressure of the melamine synthesis section. Said treatments may include quenching, purification, crystallization, solid-liquid separation and drying, so that said product stream is converted into a solid melamine product of a desired purity. Typically, melamine purification and crystallization are carried out in an alkaline environment and ammonia or sodium hydroxide are the most commonly used alkaline agents.

Irrespective of the configuration of the synthesis section and of the section of the plant treating the raw melam ine melt from the synthesis section itself, the reaction of urea to melamine produces a gaseous stream containing predominantly ammonia and carbon dioxide known as offgas, and further containing some melamine carried by the gas as well as other minor components.

Said offgas, which is liberated during the synthesis of melamine, is also termed "melamine offgas". Typically, the melamine offgas is recycled as a feed material for a tied-in urea plant. A combination of production of urea and melamine is attractive because urea is the reagent for the production of melamine and the offgas released during the synthesis of melamine contain ammonia and carbon dioxide which are the starting products for the urea synthesis. However, recycling the melamine offgas to a urea plant requires a proper purification and recover of melamine contained therein.

US 7,311,759 discloses to purify the melamine offgas with a double-stage scrubbing process. The two scrubbing stages are arranged vertically, the second stage being above the first stage. The melamine offgas is supplied to the first stage and a urea melt is introduced from above into the second stage. The urea melt traverses the two stages in countercurrent with the offgas. The contact between the offgas and the falling urea melt generates melamine precursors such as ammeline and cyanuric acid. Accordingly, a urea melt containing ammonia and melamine precursors is withdrawn from the bottom of the scrubber and partially recirculated to the first stage. The recirculated portion of urea melt is cooled in a urea melt cooler prior to reintroduction into the first stage. Cooling the urea melt recovers the heat released by the scrubbing process, particularly by the absorption of the offgas in the urea melt.

The non-recirculated portion of urea melt containing the melamine precursors is sent to the melamine synthesis section. This provides an increase in the energy efficiency of the process because less energy is required for the formation of melamine starting from said precursors rather than from pure urea melt.

Therefore, the above-described double stage scrubbing process is attractive and improves the energy efficiency of the process; however, it still has a few disadvantages.

The melamine contained in the offgas fed to the scrubber can react with said precursors, especially with cyanuric acid, to generate melamine cyanurate. Melamine cyanurate has a low solubility in urea melt under the operating temperature of the scrubber and may precipitate as melamine cyanurate, particularly in the above-mentioned urea melt cooler of the recirculation line. Said precipitation may be detrimental to the operation of the cooler. Accumulation of the melamine cyanurate may obstruct the recirculation flow and reduce the heat exchange coefficient. The decrease of the heat transfer coefficient and/or of the recirculation flow may also increase the temperature of the urea melt in the cooler and, consequently, in the first stage, which increases the risk of corrosion in the heat exchanger or in the offgas scrubber, particularly at bottom of the scrubber. The above drawbacks may render the urea melt cooler unserviceable and cause a complete shutdown of the plant.

### Summary of the invention

The invention aims to overcome the above drawbacks of the prior art. In particular the present invention aims to provide an optimized process for the synthesis of melamine, which is not subjected, or at least less sensitive, to the above drawbacks.

Accordingly, an aspect of the present invention is a process for the synthesis of melamine according to claim 1.

The process includes a two-stage purification of melamine offgas withdrawn from a melamine synthesis section. The purification process includes a first purification step performed in a first stage and a second purification step performed in a second stage. Said two steps are carried out in sequence, so that the partially purified offgas effluent from the first stage is processed in the second stage. The first purification step can be performed at a higher temperature than the second purification step, or the two steps may be performed substantially at the same temperature.

In the first purification stage, the melamine offgas is contacted (i.e., washed) with a urea melt which includes a recirculated urea melt containing ammonia and melamine precursors. Said recirculated urea melt is withdrawn from the first stage and reintroduced into the same first stage after cooling. In the second purification stage, the offgas effluent from the first stage is further contacted with a fresh urea melt.

The process of the invention is characterized in that said recirculated urea melt, prior to reintroduction into the first stage, is cooled in a shell and tube heat exchanger to a temperature equal to or above (that is, not less than) a minimum temperature of 165 °C. In certain embodiments said minimum temperature may be 170 °C or 175 °C or 180 °C. The temperature to which the recirculated urea melt is cooled is preferably in the range 165 °C to 245 °C, more preferably 170 °C to 235 °C. In certain embodiments, said temperature is in the range 175 °C to 225 °C or 180 °C to 220 °C. Preferably said temperature is the temperature of the urea melt measured at the outlet of the heat exchanger.

In a preferred embodiment, the mass ratio between said recirculated urea melt and the solid melamine product of the melamine plant, wherein the two-stage purification of the offgas is operated, is between 11 and 30. In embodiments of the invention, said mass ratio may be 13 to 28 or 14 to 22.

Said solid melamine product is the solid melamine obtained after the treatment of the raw melamine melt at a pressure lower than the synthesis pressure, for example after quenching, purification, crystallization, separation of the so obtained crystals of melamine from the remaining liquor and drying of said crystals. The mass rate of said solid melamine product normally denotes the capacity of the plant.

The selected temperature of at least 165 °C, preferably in the range 165 °C to 245 °C, to which the recirculated urea melt is cooled prior to reintroduction in the first stage, prevents the precipitation of melamine cyanurate and corrosion issues. Particularly, keeping the recirculated urea melt above 165 °C avoids the precipitation of the cyanurate, whereas a temperature not greater than 245 °C is appropriate to avoid corrosion, i.e. to control the corrosive effect of the urea melt flowing through the urea melt cooler and added to the first stage. The applicant has found that the bottom of the offgas scrubber is particularly exposed to the risk of corrosion and, thanks to the present invention, this risk is reduced. Accordingly, the risk of unwanted shutdowns of the plant is reduced, the efficiency of the purification process can be maintained in the optimal range.

The above-mentioned mass ratio between the recirculated urea melt and the amount of solid melamine produced (melamine plant capacity) provides optimal contact between urea and melamine offgas relative to size of the apparatus and power required for pumping.

A further aspect of the invention is a combined process for production of urea and melamine according to the claims.

### Description of the invention

The invention concerns a process for the synthesis of melamine. A feed stream of urea melt is reacted under non-catalytic high pressure melamine synthesis conditions to generate a raw melamine product and a stream of melamine offgas comprising ammonia, carbon dioxide, melamine and minor components. The synthesis pressure is preferably 70 bar or above, for example 70 bar to 200 bar.

Said melamine offgas is subjected to a purification process, typically to allow recycling the offgas to a tied-in urea plant. The purification process is basically a washing process (also termed scrubbing process) with a urea melt. The offgas purification process is performed at a high pressure, preferably of at least 50 bar and more preferably equal to the melamine synthesis pressure.

The purification process includes a first purification step performed in a first purification stage and a second purification step performed in a second purification stage. Said purification steps are carried out in sequence so that the melamine offgas is subject to the first purification step and the so obtained partially purified gas is further processed in the second purification step. Said first purification step is performed at a higher temperature than the second purification step.

In the first stage, the melamine offgas is contacted with a urea melt which includes a recirculated urea melt taken from the first stage itself. The urea melt which contacts the offgas in the first stage may include a urea melt previously added in the second stage and said recirculated urea melt. Said recirculated urea melt contains ammonia and melamine precursors and is cooled before reintroduction in the first stage.

According to the invention said recirculated urea melt containing ammonia and melamine precursors is cooled in a shell and tube heat exchanger to a temperature of not less than 165 °C prior to re-introduction in the first purification stage.

Preferably the recirculated urea melt is cooled in the tube side of said heat exchanger and heat removed from the urea melt is used to produce steam in the shell side of said heat exchanger. The temperature of the steam produced in the shell side is preferably between 160 °C and 240 °C, more preferably 165 °C to 230 °C.

In some embodiments the recirculated urea melt is cooled in the above-mentioned heat exchanger to a temperature of not less than 170 °C or not less than 175 °C or not less than 180 °C. Preferably said temperature is also not greater than 245 °C or not greater than 235 °C or not greater than 225 °C or not greater than 220 °C. Said temperature is preferably 165 to 245 °C or 170 to 235 °C or 175 to 225 °C or 180 to 220 °C. The lower limits and upper limits of the above-mentioned ranges may be combined. For example, further embodiments include that said temperature is 170 to 245 °C or 175 to 245 °C or 175 to 235 °C or 180 to 245 °C or 180 to 235 °C.

Depending on the temperature to which the recirculated urea melt is cooled, the temperature of the steam produced in the heat exchanger may be 170 to 220 °C or 175 to 220 °C or 170 to 215 °C or 175 to 215 °C.

Said temperature of the steam production in the shell side is selected to keep the inner surface of the tubes of the heat exchanger above the temperature at which precipitation of melamine cyanurate starts. The applicant has found that the inner surface of tubes, which is contact with the recirculated urea melt, is the point most exposed to the unwanted precipitation of melamine cyanurate. Furthermore, a melamine plant typically comprises a steam network at around 6 barg, which corresponds to 165 °C, so that the production of steam in the urea melt cooler seamlessly integrates with the steam network of the melamine plant.

The applicant has found that cooling the urea melt to not less than 165 °C is advantageous contrary to the customary practice which prompts to cool said recirculated stream as much as possible insofar the urea is above its melting temperature, to recover heat and reduce the recirculated flow rate. The applicant has judiciously found that keeping the recirculated urea melt above 165 °C reduces the drawbacks connected to precipitation of melamine cyanurate in the urea melt cooler so that the apparent disadvantage in terms of increased recirculated flowrate is well compensated.

The temperature of the shell-side steam is also in contrast to the customary approach of heat exchanger design, which would prompt to produce steam at a temperature and pressure as low as possible, to increase the difference of temperature across the urea melt cooler and make said cooler smaller.

In the second stage, a fresh urea melt is introduced. The term fresh urea melt denotes for example the urea melt obtainable from a urea plant after recovery of unreacted matter and evaporation of water. Typically, the urea melt contains at least 96% urea, the balance being residual water and unavoidable impurities.

According to a preferred embodiment, the two purification stages work with a counter-current flow. In the second stage, the melamine offgas from the first stage is contacted counter-current with a fresh urea melt; in the first stage, the melamine offgas is contacted counter-current with the urea melt from the second stage and with the recirculated urea melt containing ammonia and melamine precursors.

A preferred embodiment is as follows. The second purification stage is placed above the first purification stage; the melamine offgas flows upward in the first stage and then in the second stage; a urea melt is sprayed over the offgas from top of the second stage and flows downward through the second stage and the first stage; a urea melt loaded with ammonia and melamine precursors is collected at the bottom of the first stage and a portion thereof is recirculated to the same first stage after cooling-. Accordingly the melamine offgas is scrubbed in counter-current firstly with the urea melt from the second stage and the urea melt recirculated in the first stage; then with the fresh urea melt introduced in the second stage.

Preferably said first purification step is carried out in the temperature range 170 °C to 250 °C, more preferably in the temperature range 175 °C to 240 °C. Said second purification step is carried out preferably in the temperature range 135 °C to 230 °C. Preferably urea melt is withdrawn from the first purification stage at a temperature in the range 170 °C to 250 °C.

The urea melt withdrawn from the first stage is a mixture of fresh urea melt from the second stage and urea melt recirculated to the first stage.

The non-recirculated portion of the urea melt collected from the first stage may be added with fresh urea melt to form the feed of the melamine synthesis section. According to an embodiment, the urea melt feed stream supplied to the high-pressure melamine synthesis section includes an amount of the urea melt which is supplied to the second purification stage and an amount of the urea melt containing ammonia and melamine precursors which is withdrawn from the first purification stage. A portion of the urea melt containing ammonia and melamine precursors withdrawn from the first purification stage is recycled to the first purification stage and another portion is conveyed to the high-pressure melamine synthesis section.

The first step of purification may be performed with addition of carbon dioxide in the first stage. In an embodiment, a carbon dioxide stream is added to the melamine offgas which is conveyed to the first purification stage. In another embodiment a carbon dioxide stream is introduced directly into the first purification stage.

According to a preferred embodiment, the synthesis of melamine includes a conversion step and a stripping step, wherein said conversion step includes reacting said urea melt feed stream under suitable melamine synthesis conditions to generate a raw melamine product, and said stripping step includes the stripping of said raw melamine product in the presence of gaseous ammonia, to remove carbon dioxide contained in the raw melamine.

The conversion of urea into melamine is performed in a melamine synthesis section. In some embodiments, said melamine synthesis section includes a single reactor, from which the raw melamine and the melamine offgas are withdrawn. In other embodiments, said melamine synthesis section includes a primary reactor where urea melt is reacted, followed by a secondary reactor where the melamine-containing effluent of the primary reactor is stripped with gaseous ammonia. In such embodiments, each of the primary reactor and the secondary reactor produce a respective stream of melamine offgas. Both melamine offgas streams are made predominantly of ammonia and carbon dioxide, although they may differ in composition.

The melamine offgas subject to scrubbing with urea melt, in accordance with embodiments the invention, may include only the melamine offgas stream from the primary reactor or both melamine offgas streams from the primary reactor and secondary reactor, possibly combined into a single stream. In a further embodiment, a combined reactor performs the function of the primary reactor and secondary reactor; to this purpose, said combined reactor includes a primary reaction stage and a secondary reaction stage.

According to an embodiment, the off-gas is introduced via an off-gas distributor above or below a liquid level of the urea melt containing ammonia and melamine precursors. A preferred embodiment of said offgas distributor is disclosed in US 7,311,759.

According to another embodiment, the purification process is carried out in a scrubber and said urea melt, prior to be injected into the second stage, is divided into a plurality of sub-streams and introduced at multiple locations e.g. at multiple heights in the second stage of said scrubber.

In a combined urea-melamine embodiment, ammonia and carbon dioxide are reacted to form a urea solution in a urea synthesis section, the urea solution is processed in at least one recovery section to obtain a purified urea solution and water is removed from the solution to form a urea melt. Said urea melt is used in the above-described process for synthesis of melamine. The melamine offgas generated during the synthesis of melamine is recycled to the production of urea.

The melamine synthesis is performed at a synthesis pressure which is typically above 70 bar, such as 70 to 200 bar and preferably 75 to 200 bar. The purification of the melamine offgas is performed at a pressure up to the synthesis pressure, typically in the range 50 to 200 bar. Preferably, the purification of the melamine offgas is performed substantially at synthesis pressure. Particularly, the offgas purification process may be performed at a pressure slightly less than the melamine synthesis pressure, wherein the difference is not more than 20 bar or not more than 5 bar.

### Examples

Table 1 summarizes several experimental tests with a different temperature of the recirculated urea melt after cooling, measured at the outlet of the heat exchanger. For each test the expected lifetime of the heat exchanger and the scrubber (bottom part) was estimated and the occurrence of melamine cyanurate precipitation in the heat exchanger was also identified.

In the experimental tests according to cases 1 to 3 and 5 to 7, wherein the temperature of the recirculated urea melt was within the range 165 °C to 245 °C, no precipitation of melamine cyanurate occurred in the heat exchanger and an expected lifetime of the heat exchanger greater than 15 years was estimated. In the comparative test of case 4, wherein the temperature of the recirculated urea melt at the outlet of the heat exchanger was 158 °C, fouling of the heat exchanger was clearly detected. The shell side steam temperature was higher than 160 °C in the cases 1 to 3 and 5 to 7 and lower than 160°C in the comparative case 4.

The fresh urea inlet and the urea circulating in the first stage are given in terms of mass of urea per mass of solid melamine obtained in the process.

**Table 1**

| | | Case 1 | | Case 2 | | Case 3 | | Case 4 (comparative) | |
|---|---|---|---|---|---|---|---|---|---|
| Temperature [°C] | | Offgas | Urea | Offgas | Urea | Offgas | Urea | Offgas | Urea |
| | Inlet | 370 | 150 | 370 | 150 | 370 | 150 | 370 | 150 |
| | Transition first/second stage | 242 | 205 | 225 | 195 | 225 | 195 | 225 | 195 |
| | Outlet | 205 | 242 | 195 | 225 | 195 | 225 | 195 | 225 |
| Specific heat for steam production [kJ/mol urea] | | 24 | | 29 | | 29 | | 29 | |
| Content of melamine precursors and condensed melamine in the urea melt fed to the melamine reactor | | 7% | | 6% | | 6% | | 6% | |
| Fresh urea inlet [t_{UREA}/t_{MELAMINE}] | | 3.1 | | 3.1 | | 3.1 | | 3.1 | |
| Circulating urea in the first stage [t_{UREA}/t_{MELAMINE]} | | 20.5 | | 20.5 | | 32.0 | | 8.0 | |
| Temperature of recirculated cooled urea melt [°C] | | 220 | | 199 | | 208 | | 158 | |
| Melamine removal from the offgas (efficiency) | | 100% | | 100% | | 100% | | 95% | |
| Pumping energy ratio | | 1.0 | | 1.0 | | 1.6 | | - | |
| Melamine cyanurate precipitation in the heat exchanger | | No | | No | | No | | Yes | |
| Expected equipment lifetime (years) | | 15-20 | | >=20 | | >=20 | | - | |

**Table 1 (continues)**

| | | Case 5 | | Case 6 | | Case 7 | |
|---|---|---|---|---|---|---|---|
| Temperature [°C] | | Offgas | Urea | Offgas | Urea | Offgas | Urea |
| | Inlet | 370 | 150 | 370 | 150 | 370 | 150 |
| | Transition first/second stage | 205 | 183 | 225 | 195 | 205 | 183 |
| | Outlet | 183 | 205 | 195 | 225 | 183 | 205 |
| Specific heat for steam production [kJ/mol urea] | | 35 | | 29 | | 35 | |
| Content of melamine precursors and condensed melamine in the urea melt fed to the melamine reactor | | 4.5% | | 6% | | 4.5% | |
| Fresh urea inlet [t_{UREA}/t_{MELAMINE}] | | 3.1 | | 3.1 | | 3.1 | |
| Circulating urea in the first stage [t_{UREA}/t_{MELAMINE}] | | 20.5 | | 14.0 | | 28.0 | |
| Temperature of recirculated cooled urea melt [°C] | | 173 | | 187 | | 182 | |
| Melamine removal from the offgas (efficiency) | | 100% | | 100% | | 100% | |
| Pumping energy ratio | | 1.0 | | 0.7 | | 1.4 | |
| Melamine cyanurate precipitation in the heat exchanger | | No | | No | | No | |
| Expected equipment lifetime (years) | | >=20 | | >=20 | | >=20 | |

### Description of the figures

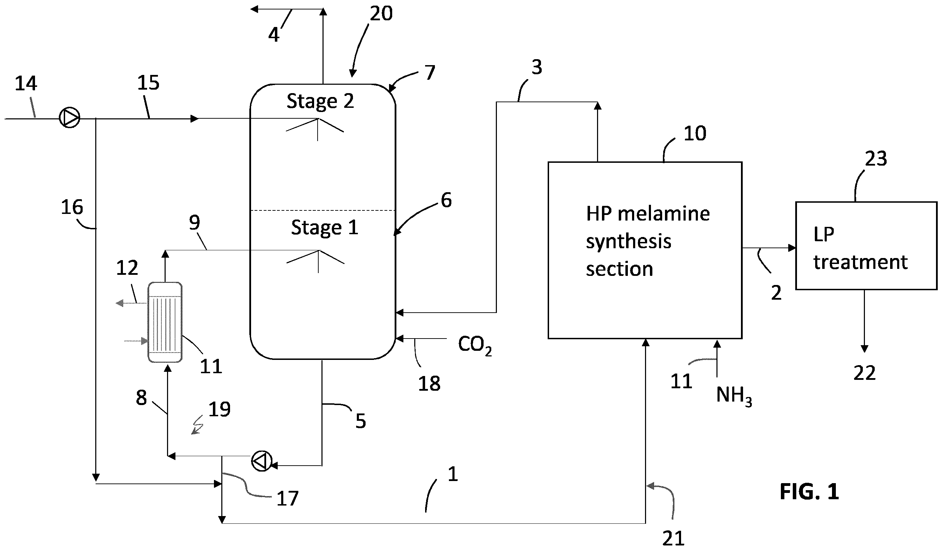

Fig. 1 is a schematic representation of a melamine synthesis process according to an embodiment of the invention.

The figure illustrates a high-pressure melamine synthesis section 10 and a melamine offgas purification section comprising a scrubber 20.

The high-pressure melamine synthesis section 10 is supplied with a urea melt feed stream 1 and gaseous ammonia 11.

In the melamine synthesis section 10 the urea feed stream 1 is reacted under high-pressure synthesis conditions to generate a raw melamine product 2 and melamine offgas 3. Said melamine offgas 3 contains carbon dioxide, ammonia, some residual melamine and other minor components. Ammonia 11 is injected in the synthesis section 10 to act as a stripping agent to remove carbon dioxide from the raw melamine.

The synthesis section 10 may comprise two separate reactors wherein in the first reactor raw melamine is synthesized and in the second reactor ammonia is used as a stripping agent to remove the carbon dioxide from the raw melamine. Alternatively, the synthesis of raw melamine and stripping with ammonia can be carried out in a single reactor. In a preferred embodiment, a single reactor has coaxial zones for synthesis and stripping. For example, the synthesis is carried out in a central zone of the reactor and stripping is carried out in an annular zone wrapped around said central zone.

The melamine offgas 3 is sent to a scrubber 20. Said scrubber 20 comprises a first stage 6 and a second stage 7. The first stage 6 is in fluid communication with the second stage 7 so that the melamine offgas 3 enters the first stage 6 and the partially purified offgas obtained from said first stage 6 are further treated in the second stage 7. Preferably, the second stage 7 is above the first stage 6 as illustrated.

The first stage 6 receives, in addition to the melamine offgas 3, a stream of gaseous carbon dioxide 18. The second stage 7 receives a fresh urea melt 15 which is a portion of a urea melt 14 coming from a tied-in urea plant (which is not shown in the figure). Said urea melt 15 is distributed, for example sprayed, from top of the second stage 7.

The scrubber 20 is traversed in counter-current by the upward flowing offgas and downward by the flowing urea melt.

Effluents of the scrubber 20 are a purified offgas 4 and a urea melt 5 containing ammonia and melamine precursors. The purified offgas 4 is withdrawn from top of the second stage 7, whereas said urea melt 5 containing ammonia and melamine precursors is collected from bottom of the first stage 6. Once extracted, said urea melt 5 is separated into a first portion 8 and a second portion 17. The first portion 8 is recycled to the first purification stage 6 after cooling in a shell-and-tube heat exchanger 11. Particularly the urea melt is recirculated via a recirculation line 19 including said heat exchanger 11. The cooled urea melt 9 leaves the heat exchanger 11 at a temperature in the range 165 °C to 245 °C.

The shell side of the heat exchanger 11 produces steam 12 with heat removed from the first portion of recirculated urea melt 8. Preferably the temperature of said steam 12 is between 160 °C and 240 °C. Particularly preferably, said steam is saturated steam at least 6 barg.

The scrubber works as follows: in the first stage 6, the ascending stream of melamine offgas 3 is washed and purified by counter-current contact with the recirculated and cooled urea melt 9, which contains ammonia and melamine precursors, and with the urea melt 15 descending from the second stage 7. The carbon dioxide 18 injected into the first stage 6, promotes the formation of the melamine precursors contained in the urea melt 5. The purified offgas 4 emerging from the second stage 7 can be recycled to a urea plant not shown in the figure, for example to the urea plant which produces the urea melt 14.

The second portion 17 of the urea melt 5, that is the portion which is not recirculated to the first purification stage 6, is mixed with an amount 16 of the urea melt 14 to form the urea melt feed stream 1.

The raw melamine melt 2 is processed in a low-pressure section 23 to obtain solid melamine 22 of a desired purity. Said section 23 preferably includes quenching, purification, crystallization, solid-liquid separation and drying.

## Claims

1. A process for the synthesis of melamine including the steps of:
reacting a feed stream (1) of urea melt under non-catalytic high pressure melamine synthesis conditions to generate a raw melamine product (2) and an offgas (3) comprising ammonia and carbon dioxide,
subjecting said offgas (3) to a purification process by washing the offgas with urea melt, to obtain a purified offgas (4) and a urea melt (5) containing ammonia and melamine precursors, wherein the purification process includes a first purification step performed in a first purification stage (6) and a second purification step performed in a second purification stage (7),
wherein said purification steps are carried out in sequence so that the offgas (3) from the melamine synthesis is subject to the first purification step and the so obtained partially purified gas is further processed in the second purification step,
wherein:
during said first purification step, the offgas (3) is contacted with urea melt comprising a recirculated urea melt (8) containing ammonia and melamine precursors, said recirculated urea melt being withdrawn from the first stage (6) and reintroduced in the same first stage,
**characterized in that** said recirculated urea melt (8), before being reintroduced in the first stage (6), is cooled in a shell and tube heat exchanger (11) to a temperature not less than 165 °C.

2. Process according to claim 1 wherein said temperature, to which the recirculated urea melt is cooled, is not less than 170 °C or not less than 175 °C or not less than 180 °C, and preferably not greater than 245 °C or not greater than 235 °C or not greater than 225 °C or not greater than 220 °C.

3. Process according to claim 1 or 2, wherein said temperature, to which the recirculated urea melt (8) is cooled, is in any one of the following ranges: 165 °C to 245 °C or 170 °C to 235 °C or 175 °C to 225 °C or 180 °C to 220 °C.

4. Process according to any of claims 1 to 3, wherein said recirculated urea melt (8) is cooled in the tube side of said heat exchanger (11) and heat removed from said urea melt is used to produce steam (12) in the shell side of said heat exchanger (11).

5. Process according to claim 4 wherein the temperature of said steam (12) produced in said shell side of the heat exchanger (11) is between 160 °C to 240 °C, preferably 165 °C to 230 °C, and preferably said steam (12) is saturated steam at a pressure of least 6 barg.

6. Process according to any of claims 1 to 5, wherein in the first purification stage (6), said offgas (3) is contacted in counter-current direction with the urea melt comprising the recirculated urea melt (8) loaded with ammonia and melamine precursors; afterward in the second stage (7), the offgas emerging form the first stage (6) is contacted in counter-current with a fresh urea melt (15); said fresh urea melt is introduced in the second stage (7) and traverses in sequence the second stage (7) and the first stage (6).

7. Process according to any of the previous claims, wherein urea melt (5) withdrawn from said first purification stage has a temperature in the range 170 °C to 250 °C, preferably 175 °C to 240 °C.

8. Process according to any of the previous claims, wherein said second purification step is carried out in the temperature range 135 °C to 230 °C.

9. Process according to any of the previous claims, wherein said raw melamine melt (2) is processed further to obtain a solid melamine product (22) and wherein the mass ratio between said recirculated urea melt (8, 9) and said solid melamine product (22) is between 11 and 30 or in the range 13 to 28 or in the range 14 to 22.

10. Process according to any of the previous claims, wherein said urea melt feed stream (1) includes fresh urea melt (16) and a portion (17) of the urea melt (5) containing ammonia and melamine precursors withdrawn from the first purification stage (6).

11. Process according to any of the previous claims, wherein carbon dioxide (18) is added to said offgas (3) in said first purification stage (6).

12. Process according to any of the previous claims, wherein the synthesis of melamine includes a conversion step and a stripping step, wherein in said conversion step said urea melt feed stream (1) is reacted under high-pressure melamine synthesis conditions to generate a raw melamine product containing carbon dioxide and in said stripping step said raw melamine product containing carbon dioxide is stripped in presence of gaseous ammonia (11).

13. Process according to claim 12, wherein: the synthesis of melamine is performed in a synthesis section (10) including a primary reactor followed by a secondary reactor, wherein said stripping step is performed in the secondary reactor, and the offgas (3) subject to said purification process includes offgas withdrawn from the primary reactor only or the offgas subject to said purification process includes offgas withdrawn from the primary reactor and offgas withdrawn from the secondary reactor, or wherein the synthesis of melamine is performed in a synthesis section including a single reactor, and the offgas subject to said purification process is withdrawn from said single reactor.

14. Process according to any of the previous claims, wherein the offgas (3) is introduced via an offgas distributor above or below a liquid level of the urea melt (5) containing ammonia and melamine precursors.

15. Process according to any of the previous claims, wherein said purification process is carried out in a scrubber (20) and a fresh urea melt (15), prior to be injected into the second stage (7), is divided into a plurality of sub-streams and introduced at multiple locations in the second stage (7) of said scrubber.

16. A combined process for the synthesis of urea and melamine wherein:
ammonia and carbon dioxide are reacted to form a urea solution in a urea synthesis section; said solution is processed in at least one recovery section to obtain a purified urea solution; water is removed from said solution to form a urea melt (14);
said urea melt is used in a process for synthesis of melamine according to any of claims 1 to 15;
melamine offgas (3) generated during the synthesis of melamine are recycled to the production of urea.

## Patentansprüche

1. Verfahren zur Synthese von Melamin, das die folgenden Schritte umfasst:
Reagieren eines Zufuhrstroms (1) von Harnstoffschmelze unter nichtkatalytischen Hochdruck-Melaminsynthesebedingungen, um ein Rohmelaminprodukt (2) und ein Abgas (3) zu erzeugen, das Ammoniak und Kohlendioxid umfasst,
Unterziehen des Abgas (3) einem Reinigungsverfahren durch Waschen des Abgases mit Harnstoffschmelze, um ein gereinigtes Abgas (4) und eine Harnstoffschmelze (5) zu erhalten, die Ammoniak- und
Melaminvorläufer enthält, wobei das Reinigungsverfahren einen ersten Reinigungsschritt umfasst, der in einer ersten Reinigungsstufe (6) durchgeführt wird, und einen zweiten Reinigungsschritt umfasst, der in einer zweiten Reinigungsstufe (7) durchgeführt wird,
wobei die Reinigungsschritte nacheinander durchgeführt werden, so dass das Abgas (3) aus der Melaminsynthese dem ersten Reinigungsschritt unterzogen wird und das so erhaltene teilweise gereinigte Gas in dem zweiten Reinigungsschritt weiterverarbeitet wird,
wobei:
während des ersten Reinigungsschritts das Abgas (3) mit Harnstoffschmelze in Kontakt gebracht wird, die eine rezirkulierte Harnstoffschmelze (8) umfasst, die Ammoniak- und Melaminvorläufer enthält, wobei die rezirkulierte Harnstoffschmelze aus der ersten Stufe (6) abgezogen und in dieselbe erste Stufe wieder eingeführt wird,
**dadurch gekennzeichnet, dass** die rezirkulierte Harnstoffschmelze (8) vor der Wiedereinführung in die erste Stufe (6) in einem Mantel- und Rohrwärmetauscher (11) auf eine Temperatur von nicht weniger als 165 °C abgekühlt wird.

2. Verfahren nach Anspruch 1, wobei die Temperatur, auf die die rezirkulierte Harnstoffschmelze abgekühlt wird, nicht weniger als 170 °C oder nicht weniger als 175 °C oder nicht weniger als 180 °C und vorzugsweise nicht mehr als 245 °C oder nicht mehr als 235 °C oder nicht mehr als 225 °C oder nicht mehr als 220 °C beträgt.

3. Verfahren nach Anspruch 1 oder 2, wobei die Temperatur, auf die die rezirkulierte Harnstoffschmelze (8) abgekühlt wird, in einem der folgenden Bereiche liegt: 165 °C bis 245 °C oder 170 °C bis 235 °C oder 175 °C bis 225 °C oder 180 °C bis 220 °C.

4. Verfahren nach einem der Ansprüche 1 bis 3, wobei die rezirkulierte Harnstoffschmelze (8) in der Rohrseite des Wärmetauschers (11) gekühlt wird und die aus der Harnstoffschmelze abgeführte Wärme zur Erzeugung von Dampf (12) in der Mantelseite des Wärmetauschers (11) verwendet wird.

5. Verfahren nach Anspruch 4, wobei die Temperatur des in der Mantelseite des Wärmetauschers (11) erzeugten Dampfes (12) zwischen 160 °C und 240 °C, vorzugsweise 165 °C und 230 °C, liegt und der Dampf (12) vorzugsweise gesättigter Dampf bei einem Druck von mindestens 6 bar(ü) ist.

6. Verfahren nach einem der Ansprüche 1 bis 5, wobei in der ersten Reinigungsstufe (6) das Abgas (3) im Gegenstrom mit der Harnstoffschmelze in Kontakt gebracht wird, die die rezirkulierte Harnstoffschmelze (8) umfasst, die mit Ammoniak- und Melaminvorläufern beladen ist; danach wird in der zweiten Stufe (7) das aus der ersten Stufe (6) austretende Abgas im Gegenstrom mit einer frischen Harnstoffschmelze (15) in Kontakt gebracht; die frische Harnstoffschmelze wird in die zweite Stufe (7) eingeführt und durchläuft nacheinander die zweite Stufe (7) und die erste Stufe (6).

7. Verfahren nach einem der vorherigen Ansprüche, wobei die aus der ersten Reinigungsstufe abgezogene Harnstoffschmelze (5) eine Temperatur im Bereich von 170 °C bis 250 °C, vorzugsweise 175 °C bis 240 °C, aufweist.

8. Verfahren nach einem der vorherigen Ansprüche, wobei der zweite Reinigungsschritt im Temperaturbereich von 135 °C bis 230 °C durchgeführt wird.

9. Verfahren nach einem der vorherigen Ansprüche, wobei die Rohmelaminschmelze (2) weiter verarbeitet wird, um ein festes Melaminprodukt (22) zu erhalten, und wobei das Massenverhältnis zwischen der rezirkulierten Harnstoffschmelze (8, 9) und dem festen Melaminprodukt (22) zwischen 11 und 30 oder im Bereich von 13 bis 28 oder im Bereich von 14 bis 22 liegt.

10. Verfahren nach einem der vorherigen Ansprüche, wobei der Harnstoffschmelze-Zufuhrstrom (1) frische Harnstoffschmelze (16) und einen Teil (17) der Harnstoffschmelze (5) umfasst, der Ammoniak- und Melaminvorläufer enthält, die aus der ersten Reinigungsstufe (6) entnommen wurden.

11. Verfahren nach einem der vorherigen Ansprüche, wobei dem Abgas (3) in der ersten Reinigungsstufe (6) Kohlendioxid (18) zugesetzt wird.

12. Verfahren nach einem der vorherigen Ansprüche, wobei die Synthese von Melamin einen Umwandlungsschritt und einen Strippingschritt umfasst, wobei in dem Umwandlungsschritt der Harnstoffschmelze-Zufuhrstrom (1) unter Hochdruck-Melaminsynthesebedingungen umgesetzt wird, um ein Rohmelaminprodukt zu erzeugen, das Kohlendioxid enthält, und in dem Strippingschritt das Rohmelaminprodukt, das Kohlendioxid enthält, in Gegenwart von gasförmigem Ammoniak (11) gestripped wird.

13. Verfahren nach Anspruch 12, wobei die Synthese von Melamin in einem Synthesebereich (10) durchgeführt wird, der einen Primärreaktor umfasst, dem ein Sekundärreaktor folgt, wobei der Strippingschritt in dem Sekundärreaktor durchgeführt wird und das Abgas (3), das dem Reinigungsverfahren unterzogen wird, nur aus dem Primärreaktor abgezogenes Abgas umfasst oder das Abgas, das dem Reinigungsverfahren unterzogen wird, aus dem Primärreaktor abgezogenes Abgas und aus dem Sekundärreaktor abgezogenes Abgas umfasst, oder wobei die Synthese von Melamin in einem Synthesebereich durchgeführt wird, der einen einzelnen Reaktor umfasst, und das dem Reinigungsverfahren unterliegende Abgas aus dem einzelnen Reaktor abgezogen wird.

14. Verfahren nach einem der vorherigen Ansprüche, wobei das Abgas (3) über einen Abgasverteiler oberhalb oder unterhalb eines Flüssigkeitsspiegels der Harnstoffschmelze (5), die Ammoniak- und Melaminvorläufer enthält, eingeleitet wird.

15. Verfahren nach einem der vorherigen Ansprüche, wobei das Reinigungsverfahren in einem Wäscher (20) durchgeführt wird und eine frische Harnstoffschmelze (15), bevor sie in die zweite Stufe (7) eingespritzt wird, in eine Vielzahl von Teilströmen aufgeteilt und an mehreren Stellen in die zweite Stufe (7) des Wäschers eingeführt wird.

16. Kombiniertes Verfahren zur Synthese von Harnstoff und Melamin, wobei:
Ammoniak und Kohlendioxid in einem Harnstoffsynthesebereich zur Bildung einer Harnstofflösung umgesetzt werden; die Lösung wird in mindestens einem Rückgewinnungsbereich verarbeitet, um eine gereinigte Harnstofflösung zu erhalten; Wasser wird aus der Lösung entfernt, um eine Harnstoffschmelze (14) zu bilden;
die Harnstoffschmelze wird in einem Verfahren zur Synthese von Melamin gemäß einem der Ansprüche 1 bis 15 verwendet;
Melaminabgase (3), die während der Synthese von Melamin entstehen, werden zur Herstellung von Harnstoff recycelt.

## Revendications

1. Procédé pour la synthèse de mélamine comportant les étapes de :
la mise en réaction d'un courant d'apport (1) de masse fondue d'urée sous des conditions de synthèse de mélamine non catalytiques à haute pression pour générer un produit de mélamine brut (2) et un gaz résiduel (3) comprenant de l'ammoniac et du dioxyde de carbone,
la soumission dudit gaz résiduel (3) à un procédé de purification par le lavage du gaz résiduel avec une masse fondue d'urée, pour obtenir un gaz résiduel purifié (4) et une masse fondue d'urée (5) contenant de l'ammoniac et des précurseurs de mélamine, dans lequel le procédé de purification comporte une première étape de purification réalisée dans un premier étage de purification (6) et une seconde étape de purification réalisée dans un second étage de purification (7),
dans lequel lesdites étapes de purification sont effectuées en séquence de sorte que le gaz résiduel (3) issu de la synthèse de mélamine soit soumis à la première étape de purification et que le gaz partiellement purifié ainsi obtenu soit ensuite traité lors de la seconde étape de purification,
dans lequel :
pendant ladite première étape de purification, le gaz résiduel (3) est mis en contact avec une masse fondue d'urée comprenant une masse fondue d'urée remise en circulation (8) contenant de l'ammoniac et des précurseurs de mélamine, ladite masse fondue d'urée remise en circulation étant prélevée du premier étage (6) et réintroduite dans le même premier étage,
**caractérisé en ce que** ladite masse fondue d'urée remise en circulation (8), avant d'être réintroduite dans le premier étage (6), est refroidie dans un échangeur de chaleur à tubes et à calandre (11) à une température qui n'est pas inférieure à 165 °C.

2. Procédé selon la revendication 1 dans lequel ladite température, à laquelle la masse fondue d'urée remise en circulation est refroidie, n'est pas inférieure à 170 °C ou n'est pas inférieure à 175 °C ou n'est pas inférieure à 180 °C, et de préférence n'est pas supérieure à 245 °C ou n'est pas supérieure à 235 °C ou n'est pas supérieure à 225 °C ou n'est pas supérieure à 220 °C.

3. Procédé selon la revendication 1 ou 2, dans lequel ladite température, à laquelle la masse fondue d'urée remise en circulation (8) est refroidie, est dans l'une quelconque des plages suivantes : de 165 °C à 245 °C ou de 170 °C à 235 °C ou de 175 °C à 225 °C ou de 180 °C à 220 °C.

4. Procédé selon l'une quelconque des revendications 1 à 3, dans lequel ladite masse fondue d'urée remise en circulation (8) est refroidie dans le côté tube dudit échangeur de chaleur (11) et de la chaleur retirée de ladite masse fondue d'urée est utilisée pour produire de la vapeur (12) dans le côté calandre dudit échangeur de chaleur (11).

5. Procédé selon la revendication 4 dans lequel la température de ladite vapeur (12) produite dans ledit côté calandre de l'échangeur de chaleur (11) est entre 160 °C et 240 °C, de préférence entre 165 °C et 230 °C, et de préférence ladite vapeur (12) est de la vapeur saturée à une pression d'au moins 6 barg.

6. Procédé selon l'une quelconque des revendications 1 à 5, dans lequel dans le premier étage de purification (6), ledit gaz résiduel (3) est mis en contact dans une direction à contre-courant avec la masse fondue d'urée comprenant la masse fondue d'urée remise en circulation (8) chargée d'ammoniac et de précurseurs de mélamine ; par la suite, dans le second étage (7), le gaz résiduel émergeant du premier étage (6) est mis en contact à contre-courant avec une masse fondue d'urée neuve (15) ; ladite masse fondue d'urée neuve est introduite dans le second étage (7) et traverse en séquence le second étage (7) et le premier étage (6) .

7. Procédé selon l'une quelconque des revendications précédentes, dans lequel la masse fondue d'urée (5) prélevée dudit premier étage de purification a une température dans la plage de 170 °C à 250 °C, de préférence de 175 °C à 240 °C.

8. Procédé selon l'une quelconque des revendications précédentes, dans lequel ladite seconde étape de purification est effectuée dans la plage de température de 135 °C à 230 °C.

9. Procédé selon l'une quelconque des revendications précédentes, dans lequel ladite masse fondue de mélamine brute (2) est ensuite traitée pour obtenir un produit de mélamine solide (22) et
dans lequel le rapport massique entre ladite masse fondue d'urée remise en circulation (8, 9) et ledit produit de mélamine solide (22) est entre 11 et 30 ou dans la plage de 13 à 28 ou dans la plage de 14 à 22.

10. Procédé selon l'une quelconque des revendications précédentes, dans lequel ledit courant d'apport (1) de masse fondue d'urée comporte une masse fondue d'urée neuve (16) et une partie (17) de la masse fondue d'urée (5) contenant de l'ammoniac et des précurseurs de mélamine prélevés du premier étage de purification (6).

11. Procédé selon l'une quelconque des revendications précédentes, dans lequel du dioxyde de carbone (18) est ajouté audit gaz résiduel (3) dans ledit premier étage de purification (6).

12. Procédé selon l'une quelconque des revendications précédentes, dans lequel la synthèse de mélamine comporte une étape de conversion et une étape de réextraction, dans lequel, à ladite étape de conversion, ledit courant d'apport (1) de masse fondue d'urée est mis en réaction sous des conditions de synthèse de mélamine à haute pression pour générer un produit de mélamine brut contenant du dioxyde de carbone et, à ladite étape de réextraction, ledit produit de mélamine brut contenant du dioxyde de carbone est réextrait en présence d'ammoniac gazeux (11).

13. Procédé selon la revendication 12, dans lequel : la synthèse de mélamine est réalisée dans une section de synthèse (10) comportant un réacteur primaire suivi d'un réacteur secondaire, dans lequel ladite étape de réextraction est réalisée dans le réacteur secondaire, et le gaz résiduel (3) soumis audit procédé de purification comporte un gaz résiduel prélevé du réacteur primaire uniquement ou le gaz résiduel soumis audit procédé de purification comporte un gaz résiduel prélevé du réacteur primaire et un gaz résiduel prélevé du réacteur secondaire, ou dans lequel la synthèse de mélamine est réalisée dans une section de synthèse comportant un unique réacteur, et le gaz résiduel soumis audit procédé de purification est prélevé dudit unique réacteur.

14. Procédé selon l'une quelconque des revendications précédentes, dans lequel le gaz résiduel (3) est introduit via un distributeur de gaz résiduel au-dessus ou au-dessous d'un niveau de liquide de la masse fondue d'urée (5) contenant des précurseurs d'ammoniac et de mélamine.

15. Procédé selon l'une quelconque des revendications précédentes, dans lequel ledit procédé de purification est effectué dans un épurateur (20) et une masse fondue d'urée neuve (15), avant d'être injectée dans le second étage (7), est divisée en une pluralité de courants partiels et introduite à de multiples emplacements dans le second étage (7) dudit épurateur.

16. Procédé combiné pour la synthèse d'urée et de mélamine dans lequel :
de l'ammoniac et du dioxyde de carbone sont mis en réaction pour former une solution d'urée dans une section de synthèse d'urée ; ladite solution est traitée dans au moins une section de récupération pour obtenir une solution d'urée purifiée ; de l'eau est retirée de ladite solution pour former une masse fondue d'urée (14) ;
ladite masse fondue d'urée est utilisée dans un procédé pour la synthèse de mélamine selon l'une quelconque des revendications 1 à 15 ;
des gaz résiduels de mélamine (3) générés pendant la synthèse de mélamine sont recyclés vers la production d'urée.
